# EUROPÄISCHE PATENTSCHRIFT

(11) **EP 0 863 884 B1**
(45) Veröffentlichungstag und Bekanntmachung des Hinweises auf die Patenterteilung: **04.10.2001**
(21) Anmeldenummer: 96939062.4
(22) Anmeldetag: 15.11.1996
(51) Int. Cl.: C07D 251/42, C09K 13/08

(54) **VERFAHREN ZUR HERSTELLUNG VON BIS-ALKOXY-TRIAZINYL-AMINOHALTIGEN STILBEN-DISULFONSÄUREN ODER DEREN DERIVATE**
PROCESS FOR PREPARING BIS-ALKOXY-TRIAZINYL-AMINO-CONTAINING STILBENE DISULPHONIC ACIDS OR THEIR DERIVATIVES
PROCEDE DE FABRICATION D'ACIDES STILBENE-DISULFONIQUES CONTENANT DU BIS-ALKOXYTRIAZINYLAMINO OU DE LEURS DERIVES

(30) Priorität: 28.11.1995 DE 19544269
(43) Veröffentlichungstag der Anmeldung: 16.09.1998
(73) Patentinhaber: BAYER AG, 51368 Leverkusen (DE)
(72) Erfinder: FELDHUES, Ulrich, D-51465 Bergisch Gladbach (DE); VOGT, Uwe, D-40789 Monheim (DE); ECKSTEIN, Udo, D-51061 Köln (DE); BROCKMANN, Rolf, D-51469 Bergisch Gladbach (DE); FIEDEL, Dietmar, D-51427 Bergisch Gladbach (DE)
(86) Internationale Anmeldenummer: EP9605033
(87) Internationale Veröffentlichungsnummer: WO9719937

(56) Entgegenhaltungen:
- DE-A- 1 444 015
- DE-A- 2 335 570
- US-A- 3 682 907
- US-A- 3 951 965
- US-A- 4 466 900

## Beschreibung

Die Erfindung betrifft ein Verfahren zur Herstellung von bis-alkoxy-triazinyl- aminohaltigen Stilben-disulfonsäuren oder deren Derivaten und ihre Verwendung als optische Aufheller für organisches Material, vornehmlich für Papier und Cellulose, insbesondere für Photopapier.

Optische Aufheller auf Basis substituierter bis-triazinylamino-stilben-2,2'-disulfonsäuren, bei denen jeweils jeder Triazinrest neben der Substitution durch die Flavonsäure und ein weiteres Amin mit einem Alkoxyrest substituiert ist, und ein Verfahren zur ihrer Herstellung sind in großer Zahl bekannt.

Der Alkoxyrest wird normalerweise nur in der ersten bzw. in der zweiten Stufe in Gegenwart eines säurebindenden Mittels eingeführt. Man setzt also ein Alkanol mit Cyanurchlorid oder mit einem noch zwei Chloratome enthaltenden Cyanurchloridderivat um. Dabei treten erhebliche unerwünschte Nebenreaktionen durch Mehrfachumsetzung am Cyanurchlorid auf. Das Einführen einer Alkoxygruppe in der dritten Stufe, d.h. in ein nur noch ein Chloratom enthaltendes Cyanurchloridderivat, erfordert spezielle Bedingungen, wie z.B. das Arbeiten in wasserfreien Glykolen bzw. Glykolmonoalkylethern in Gegenwart des entsprechenden Natriumalkoholats (DE-A 1 444 015).

In JP-A 305 983 werden Bis-triazinylamino-stilben-2,2'-disulfonsäurederivate mit Methanol im Verhältnis 1:2 bzw. mit geringem Methanolüberschuß umgesetzt.

In DE-A-2 335 570 wird beschrieben, daß es bei der Verwendung von Glykolen bzw. Glykolmonoalkylethern möglich ist, diese mit Bis-triazinylamino-stilben-2,2'-disulfonsäurederivaten in der dritten Stufe zur Reaktion zu bringen, d.h. mit einem Cyanurchloridderivat, das nur noch ein Chloratom an jedem Triazinring trägt. Die Ausbeute dieser Verfahrensweise beträgt jedoch nur zwischen 50 und 80 % und macht das Verfahren daher unattraktiv. Es bestand somit ein Bedürfnis, nach einem Verfahren mit dem man selektiv und mit verbesserten Ausbeuten, ohne den Einsatz aufwendiger und teuerer Reagenzien exakt einen C₁-C₄-Alkoxyrest pro Cyanurchlorideinheit in die Bis-triazinylamino-stilben-2,2'-disulfonsäuren einbauen kann.

Gemäß US-A-3.951.965 werden Bis-Triazinylamino-stilbendisulfonsäurederivate beschrieben, die durch jeweils stöchiometrische Umsetzung der entsprechenden Edukte erhalten werden. Eine derartige Verfahrensweise hat aber Nachteile hinsichtlich Produktqualität und -Ausbeute.

Es wurde nun ein Verfahren zur Herstellung von bis-alkoxy-triazinyl-aminohaltigen Stilben-disulfonsäuren oder deren Derivaten gefunden, bei dem man bis-chlor-triazinyl-aminohaltige Stilben-disulfonsäuren mit einem C₁-C₄-Monoalkanol umsetzt, dadurch gekennzeichnet, daß auf 1 Mol der Bis-chlor-triazinyl-aminohaltigen Stilben-disulfonsäure oder deren Derivate wenigstens 10 Mol C₁-C₄-Monoalkanol eingesetzt werden und das C₁-C₄-Monoalkanol zusammen mit Wasser eingesetzt wird, wobei der Wassergehalt der Reaktionsmischung vorzugsweise 10-80 Gew.-%, vorzugsweise 25-60 Gew.-%, bezogen auf die Reaktionsmischung, beträgt.

In einer bevorzugten Ausführungsform werden auf 1 Mol einer bis-chlor-triazinyl- aminohaltigen Stilben-disulfonsäure bzw. deren Derivate wenigstens 20 Mol des C₁-C₄-Monoalkanols eingesetzt. Besonders bevorzugt werden 50-500 Mol, insbesondere 100-300 Mol C₁-C₄-Monoalkanol pro Mol der Bis-chlor-triazinyl-aminohaltigen Stilben-sulfonsäureverbindung eingesetzt.

Als bevorzugte C₁-C₄-Monoalkanole werden Methanol, Ethanol, Isopropanol, n-Propanol, n-Butanol, sek.-Butanol oder tert.-Butanol, insbesondere Methanol eingesetzt

Besonders bevorzugt ist das Verfahren zur Herstellung von 4,4'-Bis[6-alkoxy-1,3,5-triazin-2-yl-amino]stilben-2,2'-disulfonsäure oder deren Derivate, deren Triazinylreste jeweils in 4-Position mit Aminresten substituiert sind.

Das erfindungsgemäße Verfahren wird vorzugsweise in Gegenwart eines säurebindenden Mittels durchgeführt, wobei als säurebindendes Mittel vorzugsweise Alkalihydroxid, insbesondere Kalium- oder Natriumhydroxid verwendet wird. Das säurebindende Mittel wird vorzugsweise in einer Menge von 2 bis 6 Mol Äquivalenten pro Mol der bis-chlor-triazinyl-aminohaltigen Stilben-disulfonsäure oder deren Derivate eingesetzt, vorzugsweise in einer Menge von 3 bis 4 Mol Äquivalenten.

Das erfindungsgemäße Verfahren wird in der Regel bei Temperaturen von 0°C bis zum Siedepunkt des Reaktionsgemisches, vorzugsweise bei 15 bis 80°C durchgeführt. In einer bevorzugten Ausführungsform des erfindungsgemäßen Verfahrens werden Verbindungen der allgemeinen Formel (I) 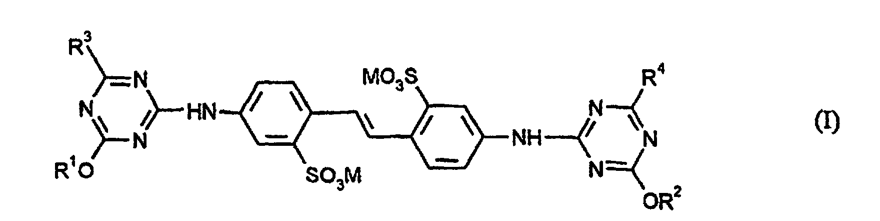 hergestellt, worin
- M: für Wasserstoff, ein Alkalimetallion oder für ein gegebenenfalls substituiertes Ammoniumion steht,
- R¹ und R²: unabhängig voneinander C₁-C₄-Alkyl bedeuten und
- R³ und R⁴: unabhängig voneinander für einen Aminrest stehen.

Die bevorzugten nach dem erfindungsgemäßen Verfahren hergestellten Verbindungen der Formel (I) werden durch Umsetzung von Verbindungen der Formel (II) 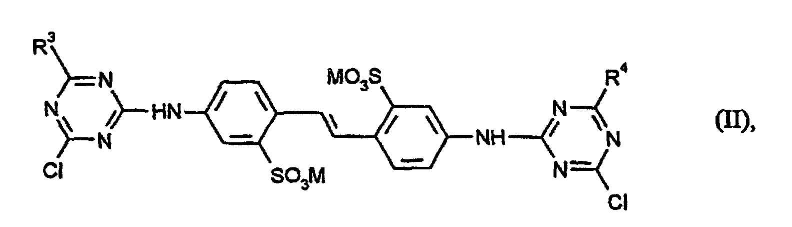 worin
- R³, R⁴: und M die oben genannten Bedeutungen haben,
mit C₁-C₄-Monoalkanolen erhalten.

Verbindungen der Formel (II) werden beispielsweise durch Umsetzung von 2 Äquivalenten Cyanurchlorid mit einem Äquivalent einer Verbindung der Formel (III) 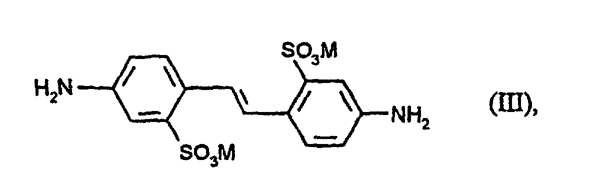 und in Summe von 2 Äquivalenten der Verbindungen R³H und R⁴H in beliebiger Reihenfolge erhalten.

Bevorzugt ist das erfindungsgemäße Verfahren, in dem die Verbindungen der Formel (II) nach ihrer Herstellung nicht zwischenisoliert werden. Besonders bevorzugt ist dabei die Verbindung der Formel (II) in Form ihrer wäßrigen Reaktionslösung, gegebenenfalls nach Aufkonzentrierung und gegebenenfalls (Teil)-Entsalzung in das erfindungsgemäße Herstellungsverfahren einzusetzen.

In einer weiteren bevorzugten Ausführungsform des erfindungsgemäßen Verfahrens, werden die Verbindungen der Formel (I) ausgehend von einer Verbindung der Formel (II) erhalten, worin R³ und R⁴ unabhängig voneinander für NR⁵R⁶ stehen, wobei R⁵ für Wasserstoff oder gegebenenfalls substituiertes C₁-C₆-Alkyl steht und R⁶ die unter R⁵ genannten Bedeutungen annehmen kann oder gegebenenfalls substituiertes C₅-C₈-Cycloalkyl oder gegebenenfalls substituiertes C₆-C₁₀-Aryl bedeutet, oder NR⁵R⁶ für gegebenenfalls durch C₁-C₄-Alkyl, insbesondere Methyl substituiertes Morpholino, Piperidino oder Hexamethylenimino steht

Bevorzugte Substituenten des C₁-C₆-Alkylrestes in der Bedeutung von R⁵ und R⁶ sind beispielsweise Hydroxy, C₁-C₆-Alkoxy, mit C₂-C₈-Alkoxy substituiertes C₂-C₈-Alkoxy, C₁-C₆-Alkylamino oder C₁-C₆-Dialkylamino, Cyano oder Sulfonsäure bzw. Sulfonat.

Als bevorzugte Substituenten des C₅-C₈-Cycloalkyls kommen in Frage: Halogen, insbesondere Fluor, Chlor und Brom, C₁-C₆-Alkyl, C₁-C₆-Alkoxy, Sulfamoyl oder Sulfonyl insbesondere C₁-C₄-Alkyl.

Als gegebenenfalls substituiertes C₆-C₁₀-Aryl kommt insbesondere sulfosubstituiertes Phenyl in Frage.

Ganz besonders bevorzugt steht R⁵ für Wasserstoff, Methyl, Sulfoethyl bzw. deren Alkalisalz oder Hydroxyethyl und R⁶ für Sulfoethyl bzw. deren Alkalisalz, Hydroxyethyl oder disulfosubstituiertes Phenyl.

In einer besonders bevorzugten Ausführungsform sind die Reste R³, R⁴ sowie R¹ und R² identisch.

Der Rest M der vorzugsweise für Wasserstoff oder ein Alkalimetall wie Natrium, Kalium oder Lithium steht, muß nicht an jeder im Molekül befindlichen Sulfonsäuregruppe identisch sein.

Weiterhin bevorzugt ist es, die nach dem erfindungsgemäßen Verfahren erhältliche Reaktionsmischung zwecks Aufarbeitung auf einen pH von kleiner als 10, vorzugsweise kleiner als 8 einzustellen, und das überschüssige C₁₋C₄-Monoalkanol, vorzugsweise destillativ zu entfernen.

Die Erfindung betrifft weiterhin Verbindungen der Formel (I), die der Formel (IV) entsprechen, 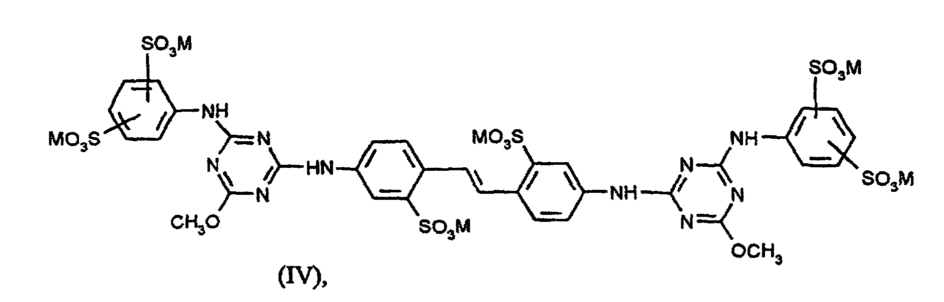 worin
- M: die oben angegebene Bedeutung besitzt.

Bevorzugte Verbindungen der Formel (IV) sind solche, in denen die SO₃M-Gruppen des terminalen Benzolringes in 2- und 5-Position stehen.

Die Verbindungen der Formel (IV) können analog der oben beschriebenen Verfahrensweise hergestellt werden.

Die nach dem erfindungsgemäßen Verfahren hergestellten Verbindungen (I) sowie die neuen Verbindungen der Formel (IV) eignen sich hervorragend als optische Aufheller für organische Materialien, vorzugsweise für Cellulose, Polyamid, Wolle, Seide und Papier, insbesondere für Photopapier. Sie können auch Waschmitteln zugesetzt werden. Die Verbindungen der Formel (I) können z.B. in Masse eingearbeitet oder auf die Oberfläche der aufzuhellenden Materialien aufgebracht werden. Besonders bevorzugte Einsatzmengen betragen 0,0001 bis 2 Gew.-%, bezogen auf aufzuhellendes Material.

Die in den nachfolgenden Beispielen angegebenen Prozentangaben sind jeweils Gewichtsprozente.

### Beispiel 1

1 000 g Methanol, welches 10% Wasser enthält, werden vorgelegt. Dann werden 185 g Natronlauge 10%ig zugesetzt. Die Temperatur wird auf 35°C eingestellt. Dann wird innerhalb 1h bei 35°C eine ca. 35°C wanne Lösung aus 167 g 4,4'-Bis[(4-(2,5-disulfo)anilino-6-chloro-1,3,5-triazin-2-yl)amino]-stilben-2,2'-disulfonsäurehexanatriumsalz in 1275 ml Wasser eingetropft. Man hält die Temperatur 2h.

Der pH wird direkt nach der Umsetzung in ca. 1h durch Zugabe von Salzsäure 10%ig auf 6,5 bis 7,0 gestellt. Danach wird solange Methanol bzw. Methanol-Wasser abdestilliert, bis ein konstanter Siedepunkt von 100°C erreicht ist. Der Ansatz wird bei ca. 30°C mit Wasser auf 1500ml aufgefüllt.
Anschließend wird die Lösung zuerst über ein Blaubandfilter und dann über einen 0,8 µm-Filter abgesaugt, um die Lösung von sämtlichen den Permeatfluß behindernden Trüb- und Schwebstoffen zu befreien. In einer Druckpermeationsanlage werden die 1500 ml eingefüllt. Bei 40 bis 50°C und ca. 40 bar werden die ersten 500 ml Permeat entzogen. Man setzt 500 ml Wasser zu und entzieht die zweiten ca. 500 ml Permeat. Es werden 500 ml Wasser zugesetzt und die dritten ca. 500 ml Permeat entzogen. Es werden 500 ml Wasser zugesetzt und die vierten ca. 500 ml Permeat entzogen. Das Konzentrat wird in einer Kristallisierschale im Vakuumtrockenschrank (50°C) zur Trockenen eingedampft und in einer Reibschale zerkleinert.

Ausbeute: 160 g gelbes Pulver 4,4'-Bis[(4-(2,5-disulfo)anilino-6-methoxy-1,3,5-triazin-2-yl)amino]-stilben-2,2'-disulfonsäurehexanatriumsalz.

Das Produkt enthält weniger als 2 % NaCl und ca. 1 % H₂O, so daß sich eine Ausbeute der Theorie von ca. 94 % ergibt.

### Beispiel 2

1 000 g Methanol, welches 5% Wasser enthält, werden vorgelegt. Dann werden 185 g Natronlauge 10%ig zugesetzt. Die Temperatur wird auf 40°C eingestellt. Dann werden innerhalb 1h bei 40°C 1 200 ml einer wäßrigen Lösung eingetragen, die ca. 65 g 4-[(4-Diethanolamino-6-chloro-1,3,5-triazin-2-yl)amino)4'-[(4-sulfoethylamino-6-chloro-1,3,5-triazin-2-yl)amino]stilben-2,2'-disulfonsäure-trinatriumsalz, 15 g 4,4'-Bis[(4-diethanolamino-6-chloro-1,3,5-triazin-2-yl)amino]stilben-2,2'-disulfonsäure-dinatriumsalz und 65 g 4,4'-Bis[(4-sulfoethylamino-6-chloro-1,3,5-triazin-2-yl)amino]stilben-2,2'-disulfonsäure-tetranatriumsalz enthält. Man hält die Temperatur 2h.

Der pH wird direkt nach der Umsetzung in ca. 1h durch Zugabe von Salzsäure 10%ig auf 6,5 bis 7,0 gestellt. Danach wird solange Methanol bzw. Methanol-Wasser abdestilliert, bis ein konstanter Siedepunkt von 100°C erreicht. ist. Der Ansatz wird bei ca. 40 bis 50°C mit Wasser auf 1 800 ml aufgefüllt Anschließend wird die Lösung zuerst über ein Blaubandfilter und dann über einen 0,8 µm-Filter abgesaugt, um die Lösung von sämtlichen den Permeatfluß behindernden Trüb- und Schwebstoffen zu befreien. In einer Druckpermeationsanlage werden die 1 800 ml eingefüllt Bei 40 bis 50°C und ca. 40 bar werden die ersten 600 ml Permeat entzogen. Man setzt 600 ml Wasser zu und entzieht die zweiten ca. 600 ml Permeat. Es werden 600 ml Wasser zugesetzt und die dritten ca. 600 ml Permeat entzogen. Es werden 600 ml Wasser zugesetzt und die vierten ca. 600 ml Permeat entzogen. Das Konzentrat wird in einer Kristallisierschale im Vakuumtrockenschrank (50°C) zur Trockenen eingedampft und in einer Reibschale zerkleinert.
Ausbeute: 140 g hellgelbes Pulver.

Das Produkt enthält weniger als 1 % NaCl und weniger als 1% Wasser, so daß sich eine Ausbeute der Theorie von 95 % ergibt

### Beispiel 3

1 000 g Methanol, welches 5% Wasser enthält, werden vorgelegt. Dann werden 185 g Natronlauge 10%ig zugesetzt. Die Temperatur wird auf 45°C eingestellt. Dann werden innerhalb 1h bei 45°C 600 ml wäßrige 45°C-warme Lösung eingetragen, die ca. 65 g 4-[(4-Diethanolamino-6-chloro-1,3,5-triazin-2-yl)amino]-4'-[(4-sulfoethylamino-6-chloro-1,3,5-triazin-2-yl)amino]stilben-2,2'-disulfonsäure-trinatriumsalz, 15 g 4,4'-Bis[(4-diethanolamino-6-chloro-1,3,5-triazin-2-yl)amino]stilben-2,2'-disulfonsäure-dinatriumsalz und 65 g 4,4'-Bis[(4-sulfoethylamino-6-chloro-1,3,5-triazin-2-yl)amino]stilben-2,2'-disulfonsäure-tetranatriumsalz enthält. Man hält die Temperatur 2h.

Der pH wird direkt nach der Umsetzung in ca. 1h durch Zugabe von Salzsäure 10%ig auf 6,5 bis 7,0 gestellt. Danach wird solange Methanol bzw. Methanol-Wasser abdestilliert, bis ein konstanter Siedepunkt von 100°C erreicht ist Der Ansatz wird bei ca. 50°C mit Wasser auf 1 200 ml aufgefüllt. Anschließend wird die Lösung zuerst über ein Blaubandfilter und dann über einen 0,8 µm-Filter abgesaugt, um die Lösung von sämtlichen den Permeatfluß behindernden Trüb- und Schwebstoffen zu befreien. In einer Druckpermeationsanlage werden die 1 200 ml eingefüllt. Bei ca. 50°C und ca. 40 bar werden die ersten 400 ml Permeat entzogen. Man setzt 400 ml Wasser zu und entzieht die zweiten ca. 400 ml Permeat Es werden 400 ml Wasser zugesetzt und die dritten ca. 400 ml Permeat entzogen. Es werden 400 ml Wasser zugesetzt und die vierten ca. 400 ml Permeat entzogen. Das Konzentrat wird in einer Kristallisierschale im Vakuumtrockenschrank (50°C) zur Trockenen eingedampft und in einer Reibschale zerkleinert.
Ausbeute: 140 g gelbes Pulver.

Das Produkt enthält weniger als 1 % NaCl und weniger als 1 % Wasser, so daß sich eine Ausbeute der Theorie von 95 % ergibt.

### Beispiel 4

1 000 g Ethanol, welches 10% Wasser enthält, werden vorgelegt Dann werden 185 g Natronlauge 10%ig zugesetzt. Die Temperatur wird auf 45°C eingestellt Dann wird innerhalb 1h bei 45°C eine ca. 45°C warme Lösung aus 167 g 4,4'-Bis[(4-(2,5-disulfo)anilino-6-chloro-1,3,5-triazin-2-yl)amino]-stilben-2,2'-disulfonsäurehexanatriumsalz in 1 275 ml Wasser eingetropft. Man hält die Temperatur 4h.

Der pH wird direkt nach der Umsetzung in ca. 1h durch Zugabe von Salzsäure 10%ig auf 6,5 bis 7,0 gestellt. Danach wird solange Ethanol bzw. Ethanol-Wasser abdestilliert, bis ein konstanter Siedepunkt von 100°C erreicht ist. Der Ansatz wird bei ca. 50°C mit Wasser auf 1500 ml aufgefüllt
Anschließend wird die Lösung zuerst über ein Blaubandfilter und dann über einen 0,8 µm-Filter abgesaugt, um die Lösung von sämtlichen den Permeatfluß behindernden Trüb- und Schwebstoffen zu befreien. In einer Druckpermeationsanlage werden die 1 500 ml eingefüllt. Bei 40 bis 50°C und ca. 40 bar werden die ersten 500 ml Permeat entzogen. Man setzt 500 ml Wasser zu und entzieht die zweiten ca. 500 ml Permeat. Es werden 500 ml Wasser zugesetzt und die dritten ca. 500 ml Permeat entzogen. Es werden 500 ml Wasser zugesetzt und die vierten ca. 500 ml Permeat entzogen.
Das Konzentrat wird in einer Kristallisierschale im Vakuumtrockenschrank (50°C) zur Trockenen eingedampft und in einer Reibschale zerkleinert.
Ausbeute: 165 g gelbes Pulver 4,4'-Bis[(4-(2,5disulfo)anilino-6-ethoxy-1,3,5-triazin-2-yl)amino]-stilben-2,2'-disulfonsäurehexanatriumsalz.

Das Produkt enthält weniger als 2 % NaCl und weniger als 2 % Wasser, so daß sich eine Ausbeute der Theorie von 93 % ergibt.

### Beispiel 5

425 g Methanol und 75 ml Wasser werden vorgelegt. Dann werden 18 g Natronlauge 45%ig zugesetzt. Dann werden 83 g 4,4'-Bis(2-anilino-6-chloro-1,3,5triazin-2-yl)amino]-stilben-2,2'-disulfonsäure-dinatriumsalz mit einem Wassergehalt von 40% eingetragen. Man erwärmt zum RF. Nach 1h RF setzt man bei ca. 70°C 10 ml gesättigte Natriumchloridlösung zu, läßt auf RT kommen, saugt ab, wäscht mit 160 g Methanol nach und trocknet bei 50°C im Vakuum.
Ausbeute: 49 g gelbes Produkt 4,4'-Bis(2-anilino-6-methoxy-1,3,5-triazin-2-yl)amino]-stilben-2,2'-disulfonsäure-dinatriumsalz, entsprechend 99,5 % der Theorie.

### Beispiel 6

800 g Methanol und 350 ml Wasser werden vorgelegt. Dann werden 81 g Natronlauge 10%ig zugesetzt. Dann werden 100 g 4,4'-Bis[(2-(4-sulfo)anilino-6-chloro-1,3,5-triazin-2-yl)amino]-stilben-2,2'-disulfonsäure-tetranatriumsalz mit einem Wassergehalt von 40% eingetragen. Man erwärmt auf 60°C und hält 1h 60°C.
Der pH wird direkt anschließend in ca. 1h durch Zugabe von Salzsäure 10%ig auf 6,5 bis 7,0 gestellt. Danach wird solange Methanol bzw. Methanol-Wasser abdestilliert, bis ein konstanter Siedepunkt von 100°C erreicht ist. Der Ansatz wird bei ca. 50°C mit Wasser auf 500ml aufgefüllt und mit 10%iger Natronlauge auf pH 9,0 gestellt.

Anschließend wird die Lösung zuerst über ein Blaubandfilter und dann über einen 0,8 µm-Filter abgesaugt, um die Lösung von sämtlichen den Permeatfluß behindernden Trüb- und Schwebstoffen zu befreien.
In einer Druckpermeationsanlage werden die 500ml eingefüllt. Bei 40 - 50°C und ca. 40 bar werden die ersten 150 ml Permeat entzogen. Man setzt 150 ml Wasser zu und entzieht die zweiten ca. 150 ml Permeat Es werden 150 ml Wasser zugesetzt und die dritten ca. 150 ml Permeat entzogen. Es werden 150 ml Wasser zugesetzt und die vierten ca. 150 ml Permeat entzogen. Das Konzentrat wird in einer Kristallisierschale im Vakuumtrockenschrank (50°C) zur Trockenen eingedampft und in einer Reibschale zerkleinert.
Ausbeute: 56 g gelbes Pulver 4,4'-Bis[(2-(4-sulfo)anilino-6-methoxy-1,3,5-triazin-2-yl)amino]-stilben-2,2'-disulfonsäure-tetranatriumsalz

Das Produkt enthält noch 1 % NaCl und 2 % Wasser, so daß sich eine Ausbeute der Theorie von 92 % ergibt.

## Patentansprüche

1. Verfahren zur Herstellung von bis-alkoxy-triazinyl-aminohaltigen Stilben-disulfonsäuren oder deren Derivaten, **dadurch gekennzeichnet, daß** bis-chlortriazinyl-aminohaltige-Stilbendisulfonsäure oder deren Derivat mit einem C₁-C₄-Monoalkanol umgesetzt wird, wobei auf 1 Mol der bis-chlor-triazinyl-aminohaltigen Stilbensulfonsäure oder deren Derivate wenigstens 10 Mol C₁-C₄-Monoalkanol eingesetzt werden und das C₁-C₄-Monoalkanol zusammen mit Wasser eingesetzt wird, wobei der Wassergehalt der Reaktionsmischung 10-80 Gew.-%, bezogen auf die Reaktionsmischung beträgt.

2. Verfahren gemäß Anspruch 1, **dadurch gekennzeichnet, daß** wenigstens 20 Mol C₁-C₄-Monoalkanol auf 1 Mol der bis-chlor-triazinyl-aminohaltigen Stilbensulfonsäure oder deren Derivate eingesetzt werden.

3. Verfahren gemäß Anspruch 1, **dadurch gekennzeichnet, daß** als C₁-C₄-Monoalkanol Methanol verwendet wird.

4. Verfahren gemäß Anspruch 1, **dadurch gekennzeichnet, daß** die Umsetzung in Gegenwart eines säurebindenden Mittels, insbesondere eines Alkalihydroxids erfolgt.

5. Verfahren gemäß Anspruch 1, **dadurch gekennzeichnet, daß** die bis-alkoxytriazinyl-aminohaltige Stilben-disulfonsäure der Formel (I) entspricht 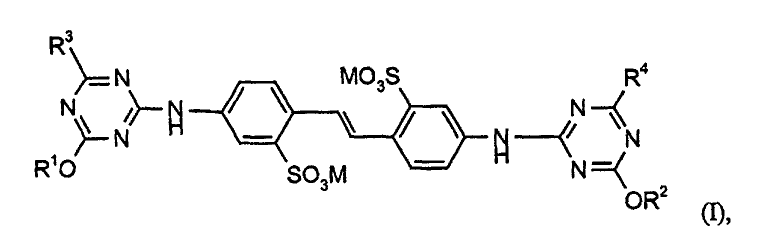 worin
M für Wasserstoff, ein Alkalimetallion oder für ein gegebenenfalls substituiertes Ammoniumion steht,
R¹ und R² unabhängig voneinander für C₁-C₄-Alkyl stehen, und
R³ und R⁴ unabhängig voneinander für einen Aminrest stehen,
und ausgehend von Verbindungen der Formel (II), 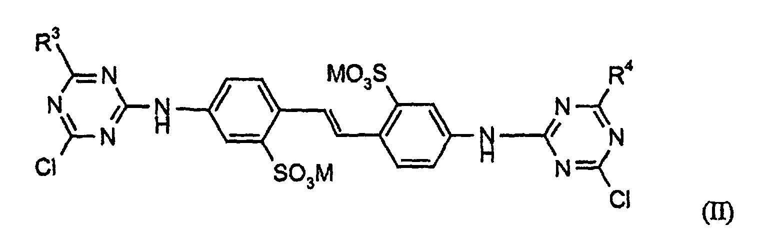 worin R³, R⁴ und M die oben genannte Bedeutung haben,
hergestellt wird.

6. Verbindungen der Formel (IV) 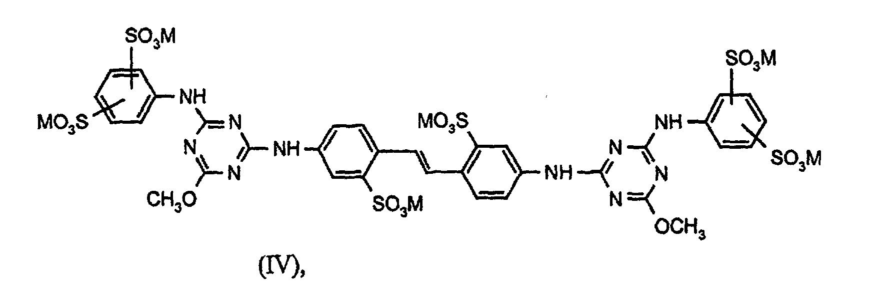 worin
M für Wasserstoff, Alkalimetall oder für gegebenenfalls substituiertes Ammonium steht.

7. Verbindungen gemäß Anspmch 6, **dadurch gekennzeichnet, daß** die beiden SO₃M-Gruppen des terminalen Benzorings in der Formel (IV) jeweils in der 2-und 5-Position stehen.

8. Verwendung von bis-alkoxy-triazinyl-aminohaltigen-Stilben-disulfonsäuren oder deren Derivate, gemäß Anspruch 6 oder 7 zum optischen Aufhellen von organischen Materialien, insbesondere von Cellulose und Papier.

9. Organisches Material, insbesondere Cellulose oder Papier, enthaltend 0,0001 bis 2 Gew.-% einer Verbindung gemäß Anspruch 6.

## Claims

1. Process for the preparation of stilbene-disulphonic acids containing bis-alkoxytriazinyl-amino, or derivatives thereof, **characterized in that** a stilbene-disulphonic acid containing bis-chloro-triazinyl-amino, or a derivative thereof, is reacted with a C₁-C₄-monoalkanol, at least 10 mol of C₁-C₄-monoalkanol being employed per mol of the stilbene-sulphonic acid containing bis-chloro-triazinyl-amino, or derivatives thereof, and the C₁-C₄-monoalkanol being employed together with water, the water content of the reaction mixture being 10-18% by weight, based on the reaction mixture.

2. Process according to Claim 1, **characterized in that** at least 20 mol of C₁-C₄-monoalkanol are employed per mol of the stilbene-sulphonic acid containing bis-chloro-triazinyl-amino, or derivatives thereof.

3. Process according to Claim 1, **characterized in that** methanol is used as the C₁-C₄-monoalkanol.

4. Process according to Claim 1, **characterized in that** the reaction is carried out in the presence of an acid-binding agent, in particular an alkali metal hydroxide.

5. Process according to Claim 1, **characterized in that** the stilbene-disulphonic acid containing bis-alkoxy-triazinyl-amino corresponds to the formula (I) 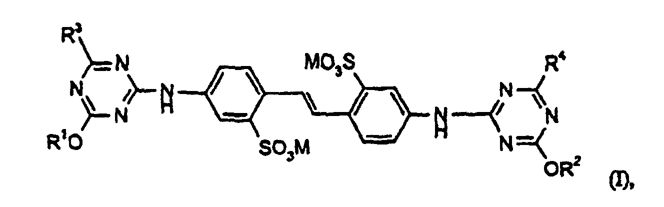 wherein
M represents hydrogen, an alkali metal ion or an optionally substituted ammonium ion,
R¹ and R² independently of one another represent C₁-C₄-alkyl and
R³ and R⁴ independently of one another represent an amine radical,
and is prepared starting from compounds of the formula (II) 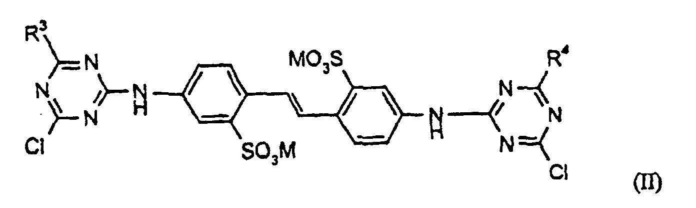 wherein R³, R⁴ and M have the abovementioned meaning.

6. Compounds of the formula (IV) 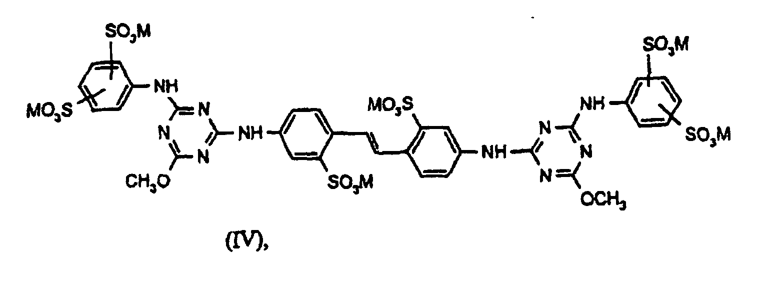 wherein
M represents hydrogen, an alkali metal or optionally substituted ammonium.

7. Compounds according to Claim 6, **characterized in that** the two SO₃M groups of the terminal benzoring in the formula (IV) are in each case in the 2- and 5-position.

8. Use of stilbene-disulphonic acids containing bis-alkoxy-triazinyl-amino, or derivatives thereof, according to Claim 6 or 7, for optical brightening of organic materials, in particular of cellulose and paper.

9. Organic material, in particular cellulose or paper, comprising 0.0001 to 2% by weight of a compound according to Claim 6.

## Revendications

1. Procédé de préparation d'acides stilbènedisulfoniques bis(alcoxytriazinylaminés) ou de leurs dérivés, **caractérisé en ce que** l'on fait réagir l'acide stilbènedisulfonique bis(chlorotriazinylaminé) ou son dérivé avec un monoalcool en C₁-C₄, où on met en oeuvre par mole d'acide stilbènesulfonique bis(chlorotriazinylaminé) ou de son dérivé, au moins 10 moles de monoalcool en C₁-C₄, et celui-ci est mis en oeuvre avec de l'eau, où la teneur en eau du mélange réactionnel se situe dans l'intervalle allant de 10 à 80% en poids, sur base du mélange réactionnel.

2. Procédé suivant la revendication 1, **caractérisé en ce que** l'on met en oeuvre au moins 20 moles de monoalcool en C₁-C₄ par mole d'acide stilbènesulfonique bis(chlorotriazinylaminé) ou de son dérivé.

3. Procédé suivant la revendication 1, **caractérisé en ce que** l'on utilise le méthanol comme monoalcool en C₁-C₄.

4. Procédé suivant la revendication 1, **caractérisé en ce que** la réaction est réalisée en présence d'un agent liant les acides, en particulier un hydroxyde alcalin.

5. Procédé suivant la revendication 1, **caractérisé en ce que** l'acide stilbènedisulfonique bis(alcoxytriazinylaminé) correspond à la formule (I) : 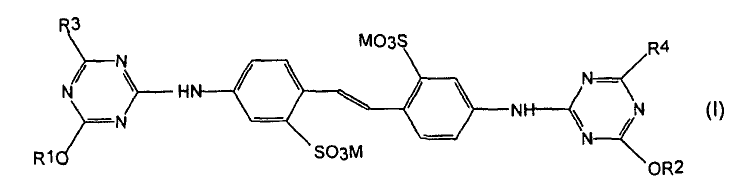 où
M représente l'atome d'hydrogène, un ion alcalin ou l'ion ammonium facultativement substitué ;
R¹ et R² représentent indépendamment l'un de l'autre, un radical alcoyle en C₁-C₄, et
R³ et R⁴ représentent indépendamment l'un de l'autre, un reste amine,
et est préparé en partant des composés de formule (II) : 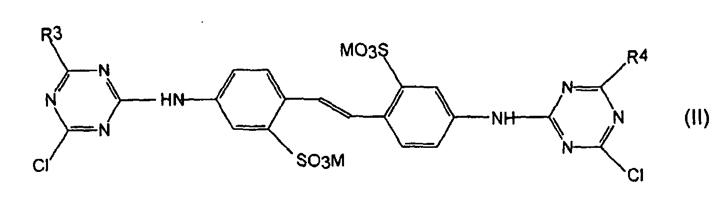 où R³, R⁴ et M ont la signification citée ci-dessus.

6. Composés de formule (IV) : 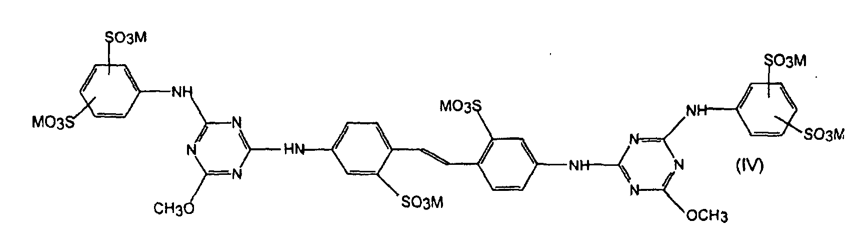 où
M représente l'atome d'hydrogène, un métal alcalin ou l'ammonium facultativement substitué.

7. Composés suivant la revendication 6, **caractérisés en ce que** les deux radicaux SO₃M des cycles benzène terminaux dans la formule (IV), se trouvent en positions 2 et 5.

8. Utilisation d'acides stilbènedisulfoniques bis(alcoxytriazinylaminés) ou de leurs dérivés suivant la revendication 6 ou 7, comme agent de blanchiment optique de matériaux organiques, en particulier de cellulose et de papier.

9. Matériau organique, en particulier cellulose ou papier, contenant 0,0001 à 2% en poids d'un composé suivant la revendication 6.
